(19) Europäisches Patentamt
European Patent Office
Office européen des brevets

(11) Veröffentlichungsnummer: **0 198 208**
**A1**

(12) # EUROPÄISCHE PATENTANMELDUNG

(21) Anmeldenummer: **86103133.4**

(22) Anmeldetag: **08.03.86**

(51) Int. Cl.⁴: **C07D 401/12** , C07D 401/14 , A61K 31/44 , A61K 31/415

(30) Priorität: **15.03.85 DE 3509333**

(43) Veröffentlichungstag der Anmeldung: **22.10.86 Patentblatt 86/43**

(84) Benannte Vertragsstaaten: **AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Rösner, Manfred, Dr.**
**Unter den Buchen 7**
**D-6239 Eppstein/Taunus(DE)**
Erfinder: **Herling, Andreas W., Dr.**
**Dieburger Strasse 43**
**D-6072 Dreieich(DE)**
Erfinder: **Bickel, Martin, Dr.**
**Mittelstedter Weg 3**
**D-6380 Bad Homburg(DE)**

(54) **Substituierte Benzimidazolderivate, Verfahren zu ihrer Herstellung, sie enthaltende pharmazeutische Zubereitungen und ihre Verwendung als Magensäuresekretionshemmer.**

(57) Benzimidazolderivate der Formel I

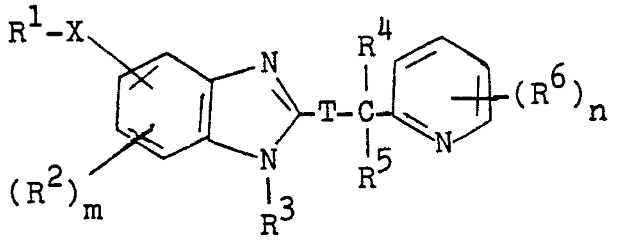

(I)

in welcher m = 3, n = 4, T -S-, -SO-oder $-SO_2-$, X -S-, -SO-, $-SO_2-$, $-SO_2-O-$, $-O-SO_2-$, $-SO_2-NR^7-$oder $-NR^7-SO_2-$und

$R^1$ einen ggf. substituierten aromatischen oder heteroaromatischen Rest, einen aliphatischen, araliphatischen oder alicyclischen Rest und $R^2$ -$R^7$ jeweils Wasserstoff oder einen organischen Rest bedeuten, sowie

Verfahren zu ihrer Hellstellung, sie enthaltende pharmazeutische Zubereitungen und ihre Verwendung als Magensäuresekretionshemmer.

EP 0 198 208 A1

# Substituierte Benzimidazolderivate, Verfahren zu ihrer Herstellung, sie enthaltende pharmazeutische Zubereitungen und ihre Verwendung als Magensäuresekretionshemmer

Benzimidazolderivate mit magensäuresekretionshemmender Wirkung sind z.B. aus DE-A-25 48 340, EP-A-5129 und DE-A-32 40 248 bekannt.

Die vorliegende Erfindung betrifft neue Benzimidazolderivate der Formel I

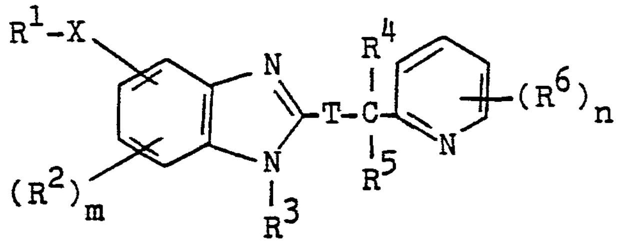

(I)

in welcher

m = 3,

n = 4,

T -S-, -SO-oder $-SO_2-$,

X -S-, -SO-, $-SO_2-$, $-SO_2-O-$, $-O-SO_2-$, $-SO_2-NR^7-$oder $-NR^7-SO_2-$und

$R^1$ einen aromatischen oder heteroaromatischen Rest mit bis zu 10 C-Atomen, der gegebenenfalls 1, 2 oder 3 gleiche oder verschiedene Substituenten aus der Reihe Halogen, Cyano, Nitro, Trifluormethyl, $(C_1-C_{12})$-Alkyl, $(C_1-C_{12})$-Alkoxy, $(C_1-C_{12})$-Alkylthio, $(C_1-C_{12})$-Alkyl-sulfinyl, $(C_1-C_{12})$-Alkylsulfonyl, ($C_3-C_8$)-Cycloalkyl, $(C_1-C_4)$-Alkanoyl, $(C_1-C_4)$-Alkanoylamino, Phenyl, Benzyl, Phenoxy, Phenylthio, Phenylsulfinyl, Phenylsulfonyl und Benzoyl trägt,

$(C_7-C_9)$-Aralkyl, $(C_3-C_8)$-Cycloalkyl, $(C_3-C_8)$-Cycloalkyl-$(C_1-C_4)$-alkyl oder, falls X <u>nicht</u> für -S- oder -SO-steht, $(C_1-C_{12})$-Alkyl bedeutet,

$R^2$ für gleiche oder verschiedene Reste aus der Reihe Wasserstoff, Halogen, Cyano, Nitro, Trifluormethyl, $(C_1-C_{12})$-Alkyl, $(C_1-C_{12})$-Alkoxy, $(C_1-C_{12})$-Alkylthio, $(C_1-C_{12})$-Alkylsulfinyl, $(C_1-C_{12})$-Alkylsulfonyl, $(C_3-C_8)$-Cycloalkyl, $(C_1-C_4)$-Alkanoyl, $(C_1-C_4)$-Alkanoylamino, Benzyl, Phenoxy, Benzoyl, $R^1$ und $R^1$-X-steht, wobei X und $R^1$ wie oben definiert sind,

$R^3$ Wasserstoff, $(C_1-C_{12})$-Alkyl, $(C_1-C_{13})$-Alkanoyl, $(C_1-C_{12})$-Alkylcarbamoyl oder eine andere physiologisch verträgliche, säurelabile $N^{im}$-Schutzgruppe bedeutet,

$R^4$ und $R^5$ gleich oder verschieden sind und Wasserstoff oder $(C_1-C_4)$-Alkyl bedeuten,

$R^6$ für gleiche oder verschiedene Reste aus der Reihe Wasserstoff, $(C_1-C_{12})$-Alkyl, $(C_1-C_{12})$-Alkoxy, $(C_1-C_{12})$-Alkoxy-$(C_1-C_{12})$-alkyl und $(C_1-C_{12})$-Alkoxy-($C_1-C_{12}$)-alkoxy steht und

$R^7$ Wasserstoff oder $(C_1-C_4)$-Alkyl bedeutet sowie deren physiologisch verträglichen Salze.

Verbindungen der Formel I sind bevorzugt, worin X $-SO_2-$, $-SO_2-O-$, $-O-SO_2-$, $-SO_2-NR^7-$oder $-NR^7-SO_2-$,

T -SO-bedeuten,

$R^2$, $R^3$, $R^4$, $R^5$ und $R^7$ jeweils Wasserstoff bedeuten, mindestens einer der Reste $R^6$ für Wasserstoff steht und die übrigen Reste wie oben definiert sind.

$R^6$ in Position 6 des Pyridin-Rings ist vorzugsweise Was serstoff, $R^6$ in Position 4 vorzugsweise $(C_1-C_6)$-Alkoxy oder $(C_1-C_6)$-Alkoxy-$(C_1-C_6)$-alkoxy.

$R^1$ kann ein aromatischer, isocyclischer Rest mit 6 -10 C-Atomen, wie Naphthyl oder, vorzugsweise, Phenyl sein. Oder $R^1$ ist ein aromatischer, heterocylischer Rest mit bis zu 10 C-Atomen (vgl. hierzu z.B. Garratt, Vollhardt, Aromatizität, Thieme Verlag Stuttgart 1973, Seiten 131 -152). Es werden darunter verstanden z.B. die Reste von Pyridin, benzoanellierten Pyridinen, 6-gliedrigen Ringen mit 2 und mehr Heteroatomen und den entsprechenden benzo anellierten Systemen, wie Pyridazin, Pyrimidin, Pyrazin, Cinnolin, Phthalazin, Chinazolin, Chinoxalin, Pteridin, Purin, Alloxazin, Triazine, 5-gliedrigen Ringen mit einem Heteroatom und den entsprechenden benzoanellierten Systemen, wie

Thiophen, Pyrrol, Furan, Indol, Isoindol und 5-gliedrigen Ringen mit 2 und mehr Heteroatomen und den entsprechenden benzoanellierten Systemen, wie Pyrazol, Imdazol, Triazol, Tetrazol, Oxazol, Thiazol, Isoxazol, Isothiazol, Indazol oder Anthranil. Bevorzugt sind Pyridyl, Thienyl, Furyl und Pyrrolyl.

Alkyl und davon abgeleitete Reste wie Alkoxy, Alkylthio, Alkanoyl usw. können geradkettig oder verzweigt sein.

Unter einer säurelabilen $N^{im}$-Schutzgruppe des Benzimidazol-Systems versteht man die schon erwähnten Alkanoyl-und Alkylcarbamoyl-Reste sowie sauer abspaltbare Gruppen vom Urethantyp wie Boc, Bpoc, Moc und Pyoc.

Gegegebenenfalls vorhandene chirale C-und S-Atome können sowohl in der R-als auch in der S-Konfiguration vorkommen. In solchen Fällen liegen Verbindungen der Formel I in Form der reinen Enantiomeren oder als Stereoisomerengemisch - (wie Enantiomerengemisch und Diastereomerengemisch) vor.

Als Salze kommen insbesondere Alkali-und Erdalkalisalze, Salze mit physiologisch verträglichen Aminen und Salze mit anorganischen oder organischen Säuren wie z.B. HCl, HBr, $H_2SO_4$, Maleinsäure, Fumarsäure in Frage.

Die Erfindung betrifft weiterhin ein Verfahren zur Herstellung von Verbindungen der Formel I, das dadurch gekennzeichnet ist, daß man

a) eine Verbindung der Formel II

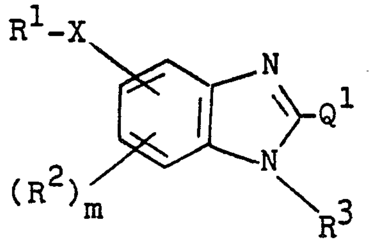

(II)

in welcher m, X, $R^1$, $R^2$ und $R^3$ wie oben definiert sind und

$Q^1$ i. eine Abgangsgruppe oder

ii. -SH, $-S^-$ oder $-SO_2^-$ bedeutet,

umsetzt mit einer Verbindung der Formel III

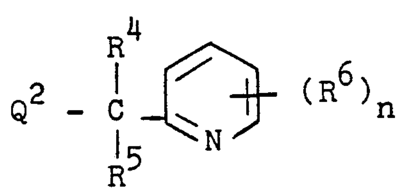

(III)

in welcher n, $R^4$, $R^5$ und $R^6$ wie oben definiert sind und $Q^2$ im obengenannten Fall i. -SH, $-S^-$ oder $-SO_2^-$, im obengenannten Fall ii. eine Abgangsgruppe bedeutet,

b) eine Verbindung der Formel IV

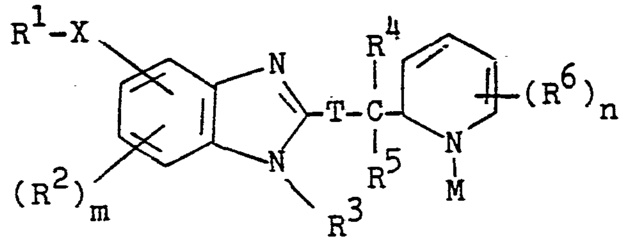

(IV)

in welcher m, n, T, X, $R^1$, $R^2$, $R^3$, $R^4$, $R^5$ und $R^6$ wie oben definiert sind und

M Wasserstoff, Li, Na, K oder MgHal und

Hal Halogen bedeutet,

Falls M Wasserstoff bedeutet, oxidiert, andernfalls erhitzt oder hydrolysiert und anschließend oxzidiert,

c) eine Verbindung der Formel V

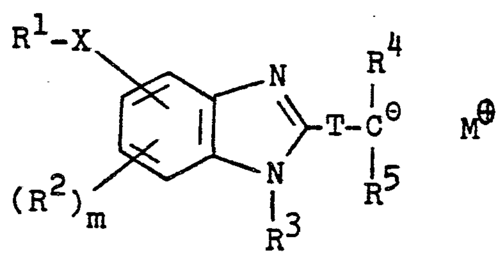

(V)

in welcher m, M, T, X, $R^1$, $R^2$, $R^3$ und $R^4$ wie oben definiert sind

mit einer Verbindung der Formel VI

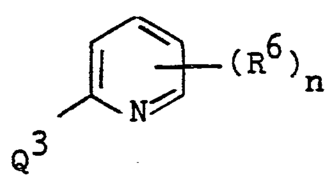

(VI)

in welcher n und $R^6$ wie oben definiert sind und $Q^3$ eine Abgangsgruppe, wie Cl oder Br bedeutet, umsetzt, oder

d) eine Verbindung der Formel VII

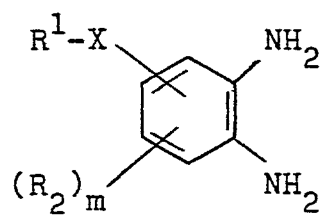

(VII)

in welcher m, X, $R^1$ und $R^2$ wie oben definiert sind, umsetzt mit einer Verbindung der Formel VIII

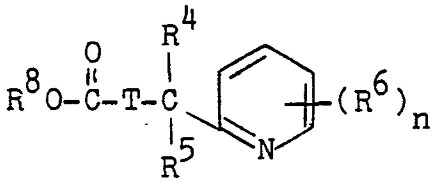

(VIII)

in welcher n, T, $R^4$, $R^5$ und $R^6$ wie oben definiert sind und

$R^8$ für eine veresternde Gruppe steht,

in den nach a) -d) erhaltenen Verbindungen der Formel I, in welcher T für -S-oder -SO-und/oder X für -S-oder -SO-steht, gegebenenfalls T und/oder X zur entsprechenden -SO-oder $-SO_2$-Gruppe oxidiert,

eine Verbindung der Formel I, worin $R^3$ für Wasserstoff steht, gegebenenfalls in Position 1 des Benzimidazol-Systems alkyliert oder acyliert,

eine Verbindung der Formel I, worin $R^3$ nicht Wasserstoff oder Alkyl bedeutet, gegebenenfalls verseift und

eine Verbindung der Formel I gegebenenfalls in ihr physiologisch verträgliches Salz überführt, wobei die Reihenfolge der letztgenannten vier Schritte auch umkehrbar ist.

Setzt man gemäß der hier bevorzugten Verfahrensvariante a) Verbindungen der Formel II mit Verbindungen der Formel III um, so steht $Q^1$ oder $Q^2$ für eine Abgangsgruppe, die nucleophil ablösbar ist, wie Cl, Br, I, $-OSO_2CH_3$, $-OSO_2CF_3$ oder $-OSO_2C_6H_4$-$pCH_3$.

Die Umsetzung einer Verbindung der Formel II mit einer Verbindung der Formel III oder deren Salzen erfolgt in einem inerten Lösungsmittel wie z.B. Wasser, Methanol, Ethanol, Aceton, Methylenchlorid, Essigsäureethylester, Toluol, Tetrahydrofuran, Acetonitril, Dimethylformamid, Dimethylsulfoxid oder Gemischen dieser Lösungsmittel, zweckmäßigerweise in Gegenwart einer anorganischen oder organischen Base wie z.B. Natrium- oder Kalium-hydroxid, -carbonat, -alkoxid, -hydrid, -amid, Ammoniak, Triethylamin, Tributylamin, Pyridin bei -20 bis +150°C vorzugsweise bei 0 -80°C.

Steht $Q^1$ oder $Q^2$ für $-SO_2^-$, so stellt man Alkali- vorzugsweise Natriumsulfinate der Formel II bzw. III her, die man in wäßriger oder alkoholische Lösung bei Wasserbadtemperatur mit Verbindungen der Formel III bzw. II, worin $Q^2$ oder $Q^1$ eine der oben definierten Abgangsgruppen, vorzugsweise Halogen wie Chlor, Brom oder Jod bedeutet, umsetzt - (vgl. z.B. J. Amer. Chem. Soc. 52 [1930] 2067).

Verbindungen der Formel II sind literaturbekannt oder können in Analogie zu bekannten Verfahren hergestellt werden, z.B. durch Ringschluß entsprechend substituierter o-Phenylendiamine mit Schwefelkohlenstoff (z.B. DE-OS 31 32 167).

Die hierfür benötigten o-Phenylendiamine sind ebenfalls literaturbekannt oder können in Analogie zu bekannten Verfahren hergestellt werden. Sie werden z.B. durch katalytische Reduktion entsprechend substituierter o-Nitroaniline erhalten.

Letztere werden im Falle von X= S z.B. durch Austausch von 5-Chlor-2-nitroanilin mit entsprechenden Thiolen gewonnen. Durch Oxidation sind Verbindungen mit X= SO und $SO_2$ zugänglich.

Sulfonsäureester (X= $-SO_2$-O-und $-O-SO_2$-) sowie Sulfonamide (X= $-SO_2-NR^4$-und $-NR^4-SO_2$-) werden aus den jeweils entsprechend substituierten Sulfonsäurehalogeniden durch Umsetzung mit den entsprechend substituierten Phenolen bzw. Anilinen erhalten.

Verbindung der Formel III sind ebenfalls literaturbekannt oder können in Analogie zu bekannten Verfahren hergestellt werden.

Verbindungen der Formel IV mit M= Li oder MgHal (wie MgJ, MgBr oder MgCl) erhält man durch Umsetzung substituierter Pyridine der Formel IX

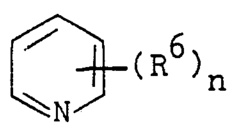

(IX)

worin n und $R^6$ wie oben definiert sind mit einer Verbindung der Formel V, worin m, M, T, X, $R^1$, $R^2$, $R^3$, $R^4$ und $R^5$ wie oben definiert ist, in einem inerten Lösungsmittel wie Diethylether oder THF bei oder unterhalb des Siedepunktes der Reaktionsmischung. Beim Erhitzen spalten die so erhaltenen Verbindungen der Formel IV MH unter Bildung von Verbindungen der Formel I ab (vgl. Klingsberg, Pyridine and Its Derivatives, Part 2, New York 1961, Seite 171). Die Hydrolyse der obengenannten Tetrahydropyridin-Derivate der Formel IV führt zu Verbindungen der Formel IV mit M = H, die spontan an der Luft oder in Gegenwart von Oxidationsmitteln, wie Nitrobenzol oder HgO in Ethanol zu Verbindungen der Formel I oxidiert werden (vgl. Kharasch, Reinmuth, Grignard Reaction of Nonmetallic Substances, Englewood Cliffs N.J. 1954, Seiten 1251 -1259).

In den bei Verfahrensvariante d) eingesetzten Estern der Formel VIII steht $R^8$ für eine veresternde Gruppe vorzugsweise ($C_1$-$C_6$) -Alkyl oder Benzyl. Die Umsetzung der Verbindungen der Formel VII mit Verbindungen der Formel VIII erfolgt analog der in Preston et al., Benzimidazoles and Congeneric Tricyclic Compounds, Part I, New York 1981, Seiten 10 -13 beschriebenen Verfahrensweisen.

Die so erhaltenen Verbindungen der Formel I worin T für -S-steht können falls $R^3$ Wasserstoff bedeutet mit Basen in physiologisch verträgliche Salze umgewandelt werden.

Verbindungen der Formel I mit X und/oder T= -S-können ferner mit geeigneten Oxidationmitteln in solche mit T bzw. X= -SO-oder X bzw. T= -$SO_2$-umgewandelt werden.

Diese Reaktion erfolgt in einem geeigneten, inerten Lösungsmittel wie z.B. Methylenchlorid, Chloroform, TetrachlorKohlenstoff, 1,2-Dichlorethan, Toluol, Essigsäureethylester, Essigsäure, Trifluoressigsäure, Wasser, Methanol, Ethanol oder Gemischen derselben bei -20°C bis +150°C vorzugsweise bei -10°C bis +40°C.

Als Oxidationsmittel kommen z.B. in Betracht: Wasserstoffperoxid, Persäuren und Perester wie Peressigsäure, Trifluorperessigsäure, Monoperphthalsäure, m-Chlorperbenzoesäure und deren Ester, Ozon, Distickstofftetroxid, Jodosobenzol, N-Chlorsuccinimid, 1-Chlorbenzotriazol, Natriumhypochlorit, Kaliumperoxodisulfat, t-Butylhypochlorit, Tetrabutylammoniumperjodat oder -permanganat, Natrium-meta-perjodat, Selen-oder Mangandioxid, Cerammonnitrat, Chromsäure, Chlor, Brom, Diazabicyclo[2.2.2]octan-Bromkomplex, Dioxandibromid, Pyridiniumperbromid, Sulfurylchlorid.

Ebenso können isolierte, ggf. immobilisierte oxidierende Enzyme oder Mikroorganismen als Oxidationsmittel Anwendung finden.

Die Oxidationsmittel werden in äquimolaren Mengen, ggf. auch in einem geringen Überschuß von 5 -10 Mol-% bei der Oxidation zu T= -SO-oder auch in größerem Überschuß und/oder bei höherer Reaktionstemperatur eingesetzt wenn eine Oxidation zu T= -$SO_2$-gewünscht wird.

Die neuen Verbindungen der Formel I und ihre Salze besitzen wertvolle pharmakologische Eigenschaften.

Sie hemmen deutlich die Magensäuresekretion und weisen darüber hinaus eine ausgezeichnete Magen-und Darmschutzwirkung auf.

Unter "Magen-und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedinger Reizmagen) verstanden, die beispielsweise durch Mikroorganismen, Bakterientoxine, Medikamente - (z.B. Antiphlogistika und Antirheumatika), Chemikalien (z.B. Ethanol), Magensäure oder Streßsituationen verursacht werden können.

Aufgrund ihrer ausgezeichneten Eigenschaften sind die substituierten Benzimidazole der Formel I und ihre pharmakologisch verträglichen Salze für den Einsatz in der Human-und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Die Erfindung betrifft daher weiter die erfindungsgemäßen Verbindungen der Formel I zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere Verbindungen der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt vorteilhafterweise zwischen 0,1 und 96 % beträgt.

Welche Hilfsstoffe für die gewünschte Arzneimittelformulierungen geeignet sind, ist dem Fachmann aufgrund seines Wissens geläufig. Neben Lösemitteln, Gelbildnern, Suppositoriengrundlagen, Tabletten-Hilfsstoffen und anderen Wirkstoffträgern können beispielsweise Antioxidantien, Dis pergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale Applikation bevorzugt ist.

Im allgemeinen hat es sich in der Humanmedizin als vorteilhaft erwiesen, den oder die Wirkstoffe bei oraler Gabe in einer Tagesdosis von etwa 0,01 bis etwa 20 mg/kg Körpergewicht, gegebenenfalls in Form mehrerer, vorzugsweise 1 bis 4 Einzelgaben zur Erzielung des gewünschten Ergebnisses zu verabreichen. Bei der parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der obengenannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat;

Tranquilizer, wie Benzodiazepine, beispielsweise Diazepam; Spasmolytika, wie z.B. Bietamiverin, Camylofin; Anticholinergica, wie z.B. Oxyphencylimin, Phencarbamid; Lokalanaesthetika, wie z.B. Tetracain,

Procain; gegebenenfalls auch Fermente, Vitamine oder Aminosäuren enthalten.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige alkoholische oder ölige Lösungen. Als inerte Träger können z.B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken-oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propanol oder Glycerin, daneben auch Zuckerlösungen wie Glucose-oder Mannitlösungen, oder auch eine Mischung aus den verschiedenen genannten Lösungsmitteln.

Die nachfolgenden Beispiele sollen die erfindungsgemäßen Verfahrensweisen erläutern, ohne die Erfindung auf die hier stellvertretend genannten Substanzen zu beschränken.

Beispiel 1

5-(4-Fluorphenylsulfonyloxy)-2-(4-methoxy-3,5-dimethyl-2-pyridylmethylthio)benzimidazol

Zu 6,48 g 4-(4-Fluorphenylsulfonyloxy)-2-mercaptobenzimidazol (Fp. 232°C Zers.) [aus 4-Fluorbenzolsulfonsäure-(3,4-diaminophenylester) mit Schwefelkohlenstoff und KOH/Ethanol] und 4,44 g 4-Methoxy-3,5-dimethyl-2-picolylchlorid-hydrochlorid tropft man bei 10°C unter Rühren 7 ml Triethylamin und rührt anschließend weitere drei Stunden bei Raumtemperatur nach. Die Reaktionslösung wird unter vermindertem Druck eingeengt und mit Wasser versetzt. Der ölige Rückstand ergibt nach Umfällung aus Isopropanol/Wasser ein harzartiges Produkt; (MS - (EI): $M^+$ = 473.

Beispiel 2

5-(4-Fluorphenylsulfonyloxy)-2-(4-methoxy-3,5-dimethyl-2-pyridylmethylsulfinyl)benzimidazol

2,13 g der Verbindung des Beispiels 1 werden bei 5°C unter Rühren in 100 ml Methylenchlorid gelöst, 0,92 g m-Chlorperbenzoesäure werden portionsweise eingetragen und zehn Minuten nachgerührt. Die Reaktionsmischung wird mit

Sodalösung versetzt, die organische Phase abgetrennt, über Natriumsulfat getrocknet und einrotiert. Der Rückstand wird aus Aceton umkristallisiert, Fp. 167°C Zers.

Beispiel 3

2-(4-Methoxy-2-pyridylmethylthio)-5-phenoxysulfonylbenzimidazol

10,7 g 2-Mercapto-4-phenoxysulfonylbenzimidazol (Fp. 200°C) (aus 3,4-Diaminobenzolsulfonsäurephenylester mit Schwefelkohlenstoff und KOH/Ethanol), 7,76 g 4-Methoxy-2-picolylchlorid-hydrochlorid werden in 100 ml Isopropanol und 20 ml Wasser mit 3,2 g Natriumhydroxid zwei Stunden zum Rückfluß erhitzt und anschließend eingeengt. Man versetzt mit Wasser, extrahiert mit Essigsäureethylester. Nach dem Trocknen der organischen Phase über Natriumsulfat wird einrotiert, der Rückstand mit Wasser versetzt, abgesaugt, mit Wasser gewaschen und getrocknet; Fp. 141°C.

Beispiel 4

2-(4-Methoxy-2-pyridylmethylsulfinyl)-5-phenoxysulfonyl-benzimidazol

1,92 g der Verbindung des Beispiels 3 werden bei 5°C unter Rühren in 100 ml Methylenchlorid gelöst und portionsweise mit 0,92 g m-Chlorperbenzoesäure versetzt. Nach 15-minütigem Nachrühren wird mit Natriumcarbonatlösung versetzt und die organische Phase abgetrennt. Nach Trocknen über Natriumsulfat und einrotieren verbleibt ein Harz, das in Essigsäureethylester gelöst wird. Nach Zugabe von Diisopropylether fällt das gewünschte Produkt aus. Man saugt ab, wäscht mit Diisopropylether und trocknet unter vermindertem Druck; Fp. ab 100°C beginnende Zersetzung.

Die folgenden Verbindungen der allgemeinen Formel I werden in Analogie zu der in den Beispielen 1 -4 beschriebenen Verfahrensweise hergestellt:

In den nachfolgenden Verbindungen der Formel I bedeuten $(R^2)_m$, $R^4$ und $R^5$ jeweils Wasserstoff (siehe aber Fußnote **); $R^1X$ steht in Position 5 des Benzimidazol-Systems.

Tabelle

| Beispiel | $R^1$ | $R^3$ | $(R^6)_n$ | X | T | Fp. °C (Z=Zers.) |
|---|---|---|---|---|---|---|
| 5 | $4-F-C_6H_4$ | H | alle H | $SO_2O$ | S | Öl |
| 6 | $4-F-C_6H_4$ | H | alle H | $SO_2O$ | SO | 153 Z |
| 7 | $4-F-C_6H_4$ | H | $4-CH_3OCH_2CH_2O$ * | $SO_2O$ | S | Öl |
| 8 | $4-F-C_6H_4$ | H | $4-CH_3OCH_2CH_2O$ * | $SO_2O$ | SO | 60 Z |
| 9 | $4-F-C_6H_4$ | H | $4-CH_3O$ * | $SO_2O$ | S | 165 |
| 10 | $4-F-C_6H_4$ | H | $4-CH_3O$ * | $SO_2O$ | SO | 100 Z |
| 11 | $C_6H_5$ | H | $4-CH_3O$ * | $SO_2O$ | S | 65 |
| 12 | $C_6H_5$ | H | $4-CH_3O$ * | $SO_2O$ | SO | 100 Z |
| 13 | $C_6H_5$ | H | $3,5-(CH_3)_2,4-CH_3O$ * | $SO_2O$ | S | Öl |
| 14 | $C_6H_5$ | H | $3,5-(CH_3)_2,4-CH_3O$ * | $SO_2O$ | SO | 167 Z |
| 15 | $2,3-Cl_2-C_6H_3$ | H | alle H | $SO_2O$ | S | Harz |
| 16 | $2,3-Cl_2-C_6H_3$ | H | alle H | $SO_2O$ | SO | 150 Z |
| 17 | $3-CF_3-C_6H_4$ | H | alle H | $SO_2O$ | S | Öl |
| 18 | $3-CF_3-C_6H_4$ | H | alle H | $SO_2O$ | SO | 128 Z |
| 19 | $C_6H_5$ | H | $3,5-(CH_3)_2,4-CH_3O$ * | $OSO_2$ | S | 108 |
| 20 | $C_6H_5$ | H | $3,5-(CH_3)_2,4-CH_3O$ * | $OSO_2$ | SO | 110 Z |
| 21 | $3-CF_3C_6H_4$ | H | alle H | $OSO_2$ | S | 164 |
| 22 | $3-CF_3-C_6H_4$ | H | alle H | $OSO_2$ | SO | 60 Z |
| 23 | $C_6H_5$ | $CH_3$ | alle H | $OSO_2$ | S | 105 |
| 24 | $C_6H_5$ | $CH_3$ | alle H | $OSO_2$ | SO | 165 Z |
| 25 | $4-CH_3-C_6H_4$ | H | $4-CH_3O$ * | $SO_2O$ | S | 173 |
| 26 | $4-CH_3-C_6H_4$ | H | $4-CH_3O$ * | $SO_2O$ | SO | 108 Z |
| 27 | $4-CH_3-C_6H_4$ | H | $4-CH_3O$ * | $SO_2O$ | $SO_2$ | 110 Z |
| 28 | $3-CH_3SO_2-C_6H_4$ | H | $4-CH_3O$ * | $SO_2O$ | S | 75 |
| 29 | $3-CH_3SO_2-C_6H_4$ | H | $4-CH_3O$ * | $SO_2O$ | SO | 70 Z |

| Beispiel | $R^1$ | $R^3$ | $(R^6)_n$ | X | T | Fp. °C (Z=Zers.) |
|---|---|---|---|---|---|---|
| 30 | $4-CH_3O-C_6H_4$ | H | $4-CH_3O$ * | $SO_2O$ | S | 155 |
| 31 | $4-CH_3O-C_6H_4$ | H | $4-CH_3O$ * | $SO_2O$ | SO | 113 Z |
| 32 | $4-Cl-C_6H_4$ | H | $4-CH_3O$ * | $SO_2O$ | S | |
| 33 | $4-Cl-C_6H_4$ | H | $4-CH_3O$ * | $SO_2O$ | SO | |
| 34 | $2-F-C_6H_4$ | H | $4-CH_3O$ * | $SO_2O$ | S | 154 |
| 35 | $2-F-C_6H_4$ | H | $4-CH_3O$ * | $SO_2O$ | SO | 110 Z |
| 36 | $3,4-F_2-C_6H_3$ | H | $4-CH_3O$ * | $SO_2O$ | S | |
| 37 | $3,4-F_2-C_6H_3$ | H | $4-CH_3O$ * | $SO_2O$ | SO | |
| 38 | 3-Pyridyl | H | $4-CH_3O$ * | $SO_2O$ | S | |
| 39 | 3-Pyridyl | H | $4-CH_3O$ * | $SO_2O$ | SO | |
| 40 | 2-Thienyl | H | $4-CH_3O$ * | $SO_2O$ | S | |
| 41 | 2-Thienyl | H | $4-CH_3O$ * | $SO_2O$ | SO | |
| 42 | $4-F-C_6H_4$ | H | $4-CH_3O$ * | $SO_2O$ | $SO_2$ | |
| 43 | $C_6H_5$ | H | $4-CH_3O$ * | $SO_2NH$ | S | 95 |
| 44 | $C_6H_5$ | H | $4-CH_3O$ * | $SO_2NH$ | SO | 105 Z |
| 45 | $C_6H_5$ | H | $4-CH_3O$ * | $SO_2NCH_3$ | S | |
| 46 | $C_6H_5$ | H | $4-CH_3O$ * | $SO_2NCH_3$ | SO | |
| 47 | $4-CH_3-C_6H_4$ | H | $4-CH_3O$ * | $OSO_2$ | S | 151 |
| 48 | $4-CH_3-C_6H_4$ | H | $4-CH_3O$ * | $OSO_2$ | SO | 145 Z |
| 49 | $4-t-C_4H_9-C_6H_4$ | H | $4-CH_3O$ * | $OSO_2$ | S | |
| 50 | $4-t-C_4H_9-C_6H_4$ | H | $4-CH_3O$ * | $OSO_2$ | SO | |
| 51 | $4-CH_3O-C_6H_4$ | H | $4-CH_3O$ * | $OSO_2$ | S | 168 |
| 52 | $4-CH_3O-C_6H_4$ | H | $4-CH_3O$ * | $OSO_2$ | SO | 162 Z |
| 53 | $4-Cl-C_6H_4$ | H | $4-CH_3O$ * | $OSO_2$ | S | |
| 54 | $4-Cl-C_6H_4$ | H | $4-CH_3O$ * | $OSO_2$ | SO | |
| 55 | $4-CH_3CONH-C_6H_4$ | H | $4-CH_3O$ * | $OSO_2$ | S | |
| 56 | $4-CH_3CONH-C_6H_4$ | H | $4-CH_3O$ * | $OSO_2$ | SO | |
| 57 | $C_6H_5$ ** | H | $4-CH_3O$ * | $OSO_2$ | S | |
| 58 | $C_6H_5$ ** | H | $4-CH_3O$ * | $OSO_2$ | SO | |
| 59 | $C_6H_5$ | H | $4-CH_3O$ * | $NHSO_2$ | S | Harz |
| 60 | $C_6H_5$ | H | $4-CH_3O$ * | $NHSO_2$ | SO | |
| 61 | $C_6H_5$ | H | $4-CH_3O$ * | $NCH_3-SO_2$ | S | |
| 62 | $C_6H_5$ | H | $4-CH_3O$ * | $NCH_3-SO_2$ | SO | |
| 63 | $C_6H_5$ | H | $3,5-(CH_3)_2,4-CH_3O$ * | S | S | 103 |
| 64 | $C_6H_5$ | H | $3,5-(CH_3)_2,4-CH_3O$ * | SO | SO | 105 Z |
| 65 | $C_6H_5$ | H | $4-CH_3O$ * | $SO_2$ | S | 177 |
| 66 | $C_6H_5$ | H | $4-CH_3O$ * | $SO_2$ | SO | 180 Z |

| Beispiel | $R^1$ | $R^3$ | $(R^6)_n$ | X | T | Fp. °C (Z=Zers.) |
|---|---|---|---|---|---|---|
| 67 | $C_6H_5$ | H | $3,5-(CH_3)_2,4-CH_3O$ | $SO_2$ | S | |
| 68 | $C_6H_5$ | H | $3,5-(CH_3)_2,4-CH_3O$ | $SO_2$ | SO | |
| 69 | $C_6H_5$ | H | alle H | $SO_2$ | S | |
| 70 | $C_6H_5$ | H | alle H | $SO_2$ | SO | |
| 71 | $CH_3$ | H | $4-CH_3O$ * | $SO_2$ | S | |
| 72 | $CH_3$ | H | $4-CH_3O$ * | $SO_2$ | SO | |
| 73 | $C_3H_7$ | H | $4-CH_3O$ * | $SO_2$ | S | 146 |
| 74 | $C_3H_7$ | H | $4-CH_3O$ * | $SO_2$ | SO | 120 Z |
| 75 | $s-C_4H_9$ | H | $4-CH_3O$ * | $SO_2$ | S | |
| 76 | $s-C_4H_9$ | H | $4-CH_3O$ * | $SO_2$ | SO | |
| 77 | $C_6H_5-CH_2$ | H | $4-CH_3O$ * | $SO_2$ | S | |
| 78 | $C_6H_5-CH_2$ | H | $4-CH_3O$ * | $SO_2$ | SO | |
| 79 | $C_6H_{11}$ | H | $4-CH_3O$ * | $SO_2$ | S | |
| 80 | $C_6H_{11}$ | H | $4-CH_3O$ * | $SO_2$ | SO | |
| 81 | $C_6H_5$ | H | $4-CH_3O$ * | S | S | |
| 82 | $C_6H_5$ | H | $4-CH_3O$ * | SO | SO | |
| 83 | $4-CH_3-C_6H_4$ | H | $4-CH_3O$ * | S | S | |
| 84 | $4-CH_3-C_6H_4$ | H | $4-CH_3O$ * | SO | SO | |
| 85 | $4-Cl-C_6H_4$ | H | $4-CH_3O$ * | S | S | |
| 86 | $4-Cl-C_6H_4$ | H | $4-CH_3O$ * | SO | SO | |
| 87 | $4-iC_3H_7C_6H_4$ | H | $4-CH_3O$ * | S | S | Öl |
| 88 | $4-iC_3H_7C_6H_4$ | H | $4-CH_3O$ * | SO | SO | 75 Z |
| 89 | $4-C_6H_5O-C_6H_4$ | H | $4-CH_3O$ * | $SO_2O$ | S | 52 |
| 90 | $4-C_6H_5O-C_6H_4$ | H | $4-CH_3O$ * | $SO_2O$ | SO | 90 Z |
| 91 | $4-t-C_4H_9-C_6H_4$ | H | $4-CH_3O$ * | $SO_2O$ | S | 136 |
| 92 | $4-t-C_4H_9-C_6H_4$ | H | $4-CH_3O$ * | $SO_2O$ | SO | 80 Z |
| 93 | $3,4-Cl_2-C_6H_3$ | H | $4-CH_3O$ * | $SO_2O$ | S | 50 |
| 94 | $3,4-Cl_2-C_6H_3$ | H | $4-CH_3O$ * | $SO_2O$ | SO | 75 Z |
| 95 | $3-Cl-C_6H_4$ | H | $4-CH_3O$ * | $OSO_2$ | S | 125 |
| 96 | $3-Cl-C_6H_4$ | H | $4-CH_3O$ * | $OSO_2$ | SO | 78 Z |
| 97 | $C_4H_9$ | H | $4-CH_3O$ * | $SO_2O$ | S | Öl |
| 98 | $C_4H_9$ | H | $4-CH_3O$ * | $SO_2O$ | SO | 50 Z |

* Die restlichen Reste $R^6$ bedeuten jeweils H.

** Ein $R^2$ bedeutet 6-Cl, die restlichen jeweils H.

**Ansprüche**

1. Verbindung der Formel I

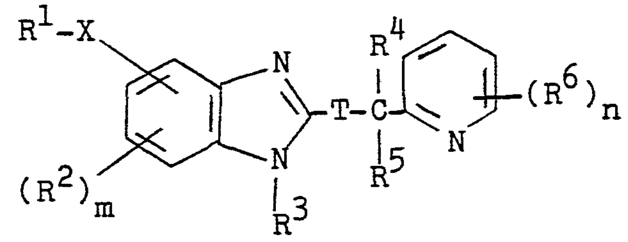

(I)

in welcher

m = 3,

n= 4

T -S-, -SO-oder -$SO_2$-,

X -S-, -SO-, -$SO_2$-, -$SO_2$-O-, -O-$SO_2$-, -$SO_2$-$NR^7$-oder -$NR^7$-$SO_2$-und

$R^1$ einen aromatischen oder heteroaromatischen Rest mit bis zu 10 C-Atomen, der gegebenenfalls 1, 2 oder 3 gleiche oder verschiedene Substituenten aus der Reihe Halogen, Cyano, Nitro, Trifluormethyl, ($C_1$-$C_{12}$)-Alkyl, ($C_1$-$C_{12}$)-Alkoxy, ($C_1$-$C_{12}$)-Alkylthio, ($C_1$-$C_{12}$)-Alkyl-sulfinyl, ($C_1$-$C_{12}$)-Alkylsulfonyl, ( $C_3$-$C_8$)-Cycloalkyl, ($C_1$-$C_4$)-Alkanoyl, ($C_1$-$C_4$)-Alkanoylamino, Phenyl, Benzyl, Phenoxy, Phenylthio, Phenylsulfinyl, Phenylsulfonyl und Benzoyl trägt,

($C_7$-$C_9$)-Aralkyl, ($C_3$-$C_8$)-Cycloalkyl, ($C_3$-$C_8$)-Cycloalkyl-($C_1$-$C_4$)-alkyl oder, falls X nicht für -S-oder -SO-steht, ($C_1$-$C_{12}$)-Alkyl bedeutet,

$R^2$ für gleiche oder verschiedene Reste aus der Reihe Wasserstoff, Halogen, Cyano, Nitro, Trifluormethyl, ($C_1$-$C_{12}$)-Alkyl, ($C_1$-$C_{12}$)-Alkoxy, ($C_1$-$C_{12}$)-Alkylthio, ($C_1$-$C_{12}$)-Alkylsulfinyl, ($C_1$-$C_{12}$)-Alkylsulfonyl, ($C_3$-$C_8$)-Cycloalkyl, ($C_1$-$C_4$)-Alkanoyl, ($C_1$-$C_4$)-Alkanoylamino, Benzyl, Phenoxy, Benzoyl, $R^1$ und $R^1$-X-steht, wobei X und $R^1$ wie oben definiert sind,

$R^3$ Wasserstoff, ($C_1$-$C_{12}$)-Alkyl, ($C_1$-$C_{13}$)-Alkanoyl, ($C_1$-$C_{12}$)-Alkylcarbamoyl oder eine andere physiologisch verträgliche, säurelabile $N^{im}$-Schutzgruppe bedeutet,

$R^4$ und $R_5$ gleich oder verschieden sind und Wasserstoff oder ($C_1$-$C_4$)-Alkyl bedeuten,

$R^6$ für gleiche oder verschiedene Reste aus der Reihe Wasserstoff, ($C_1$-$C_{12}$)-Alkyl, ($C_1$-$C_{12}$)-Alkoxy, ($C_1$-$C_{12}$)-Alkoxy-($C_1$-$C_{12}$)-alkyl und ($C_1$-$C_{12}$)-Alkoxy-( $C_1$-$C_{12}$)-alkoxy steht und

$R^7$ Wasserstoff oder ($C_1$-$C_4$)-Alkyl bedeutet sowie deren physiologisch verträglichen Salze.

2. Verbindung der Formel I gemäß Anspruch 1, in welcher

X - $SO_2$-, -$SO_2$-O-, -O-$SO_2$-, -$SO_2$-$NR^7$-oder -$NR^7$-$SO_2$-,

T -SO-bedeuten,

$R^2$, $R^3$ $R^4$, $R^5$ und $R^7$ jeweils Wasserstoff bedeuten, mindestens einer der Reste $R^6$ für Wasserstoff steht und die übrigen Reste wie oben definiert sind, sowie deren physiologisch verträglichen Salze.

3. Verbindung der Formel I gemäß Anspruch 1 oder 2, in welcher der Rest $R^6$ in Position 6 des Pyridin-Rings Wasserstoff bedeutet, sowie deren physiologisch verträglichen Salze.

4. Verbindung der Formel I gemäß Anspruch 3, in welcher der Rest $R^6$ in Position 4 des Pyridin-Rings ($C_1$-$C_6$)-Alkoxy oder ($C_1$-$C_6$)-Alkoxy-($C_1$-$C_6$)-alkoxy bedeutet, sowie deren physiologisch verträglichen Salze.

5. Verfahren zur Herstellung einer Verbindung der Formel I gemäß einem der Ansprüche 1 -4, dadurch gekennzeichnet, daß man

a) eine Verbindung der Formel II

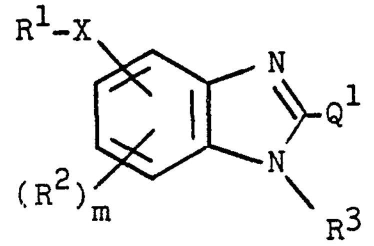

(II)

in welcher m, X, $R^1$, $R^2$ und $R^3$ wie im Anspruch 1 definiert sind und

$Q^1$ i. eine Abgangsgruppe oder

ii. -SH, $-S^-$ oder $-SO_2^-$ bedeutet,

umsetzt mit einer Verbindung der Formel III

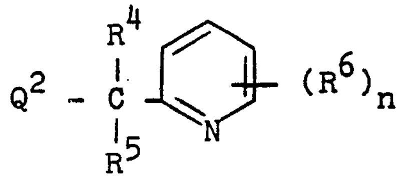

(III)

in welcher n, $R^4$, $R^5$ und $R^6$ wie im Anspruch 1 definiert sind und

$Q^2$ im obengenannten Fall i. -SH, $-S^-$ oder $-SO_2^-$, im obengenannten Fall ii. eine Abgangsgruppe bedeutet,

b) eine Verbindung der Formel IV

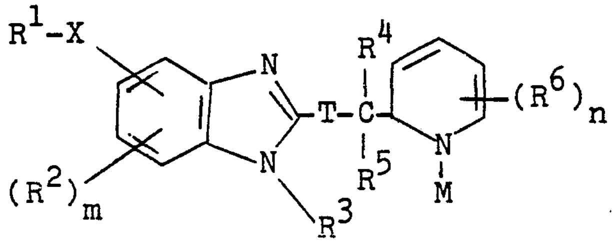

(IV)

in welcher m, n, T, X, $R^1$, $R^2$, $R^3$, $R^4$, $R^5$ und $R^6$ wie im Anspruch 1 definiert sind und M Wasserstoff, Na, K, Li oder MgHal und

Hal Halogen bedeutet,

Falls M Wasserstoff bedeutet, oxidiert, andernfalls erhitzt oder hydrolysiert und anschließend oxidiert,

c) eine Verbindung der Formel V

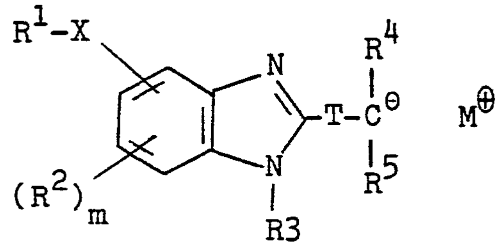

(V)

in welcher m, M, T, X, $R^1$, $R^2$, $R^3$ und $R^4$ wie oben definiert sind,

mit einer Verbindung der Formel VI

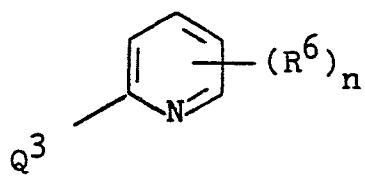

(VI)

in welcher n und $R^6$ wie oben definiert sind und $Q^3$ eine Abgangsgruppe bedeutet, umsetzt, oder

d) eine Verbindung der Formel VII

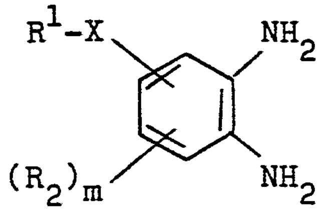

(VII)

in welcher m, X, $R^1$ und $R^2$ wie im Anspruch 1 definiert sind, umsetzt mit einer Verbindung der Formel VIII

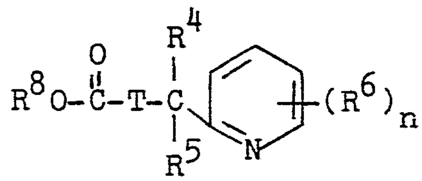

(VIII)

in welcher n, T, $R^4$, $R^5$ und $R^6$ wie im Anspruch 1 definiert sind und

$R^8$ für eine veresternde Gruppe steht,

in den nach a) -d) erhaltenen Verbindungen der Formel I, in welcher T für -S-oder -SO-und/oder X für -S-oder -SO-steht, gegebenenfalls T und/oder X zur entsprechenden -SO-oder -$SO_2$-Gruppe oxidiert,

eine Verbindung der Formel I, worin $R^3$ für Wasserstoff steht, gegebenenfalls in Position 1 des Benzimidazol-Systems alkyliert oder acyliert,

eine Verbindung der Formel I, worin $R^3$ nicht Wasserstoff oder Alkyl bedeutet, gegebenenfalls verseift und

eine Verbindung der Formel I gegebenenfalls in ihr physiologisch verträgliches Salz überführt, wobei die Reihenfolge der letztgenannten vier Schritte auch umkehrbar ist.

6. Verbindung gemäß einem der Ansprüche 1 -4 zur Anwendung als Heilmittel.

7. Verbindung gemäß einem der Ansprüche 1 -4 zur Anwendung als Magensäuresekretionshemmer.

8. Pharmazeutisches Mittel enthaltend eine Verbindung oder mehrere Verbindungen gemäß einem der Ansprüche 1 -4.

Patentansprüche Österreich:

1. Verfahren zur Herstellung einer Verbindung der Formel I

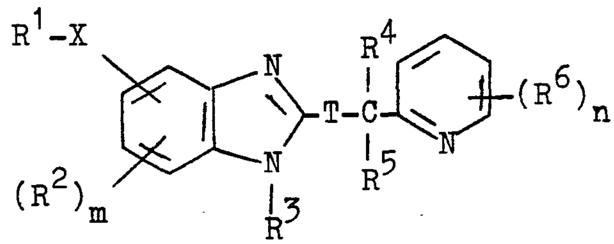

(I)

in welcher

m = 3,

n= 4,

T -S-, -SO-oder $-SO_2-$,

X -S-, -SO-, $-SO_2-$, $-SO_2-O-$, $-O-SO_2-$, $-SO_2-NR^7-$oder $-NR^7-SO_2-$und

$R^1$ einen aromatischen oder heteroaromatischen Rest mit bis zu 10 C-Atomen, der gegebenenfalls 1, 2 oder 3 gleiche oder verschiedene Substituenten aus der Reihe Halogen, Cyano, Nitro, Trifluormethyl, $(C_1-C_{12})$-Alkyl, $(C_1-C_{12})$-Alkoxy, $(C_1-C_{12})$-Alkylthio, $(C_1-C_{12})$-Alkyl-sulfinyl, $(C_1-C_{12})$-Alkylsulfonyl, ( $C_3-C_8$)-Cycloalkyl, $(C_1-C_4)$-Alkanoyl, $(C_1-C_4)$-Alkanoylamino, Phenyl, Benzyl, Phenoxy, Phenylthio, Phenylsulfinyl, Phenylsulfonyl und Benzoyl trägt,

$(C_7-C_9)$-Aralkyl, $(C_3-C_8)$-Cycloalkyl, $(C_3-C_8)$-Cycloalkyl-$(C_1-C_4)$-alkyl oder, falls X nicht für -S- oder -SO-steht, $(C_1-C_{12})$-Alkyl bedeutet,

$R^2$ für gleiche oder verschiedene Reste aus der Reihe Wasserstoff, Halogen, Cyano, Nitro, Trifluormethyl, $(C_1-C_{12})$-Alkyl, $(C_1-C_{12})$-Alkoxy, $(C_1-C_{12})$-Alkylthio, $(C_1-C_{12})$-Alkylsulfinyl, $(C_1-C_{12})$-Alkylsulfonyl, $(C_3-C_8)$-Cycloalkyl, $(C_1-C_4)$-Alkanoyl, $(C_1-C_4)$-Alkanoylamino, Benzyl, Phenoxy, Benzoyl, $R^1$ und $R^1$-X-steht, wobei X und $R^1$ wie oben definiert sind,

$R^3$ Wasserstoff, $(C_1-C_{12})$-Alkyl, $(C_1-C_{13})$-Alkanoyl, $(C_1-C_{12})$-Alkylcarbamoyl oder eine andere physiologisch verträgliche, säurelabile $N^{im}$-Schutzgruppe bedeutet,

$R^4$ und $R^5$ gleich oder verschieden sind und Wasserstoff oder $(C_1-C_4)$-Alkyl bedeuten,

$R^6$ für gleiche oder verschiedene Reste aus der Reihe Wasserstoff, $(C_1-C_{12})$-Alkyl, $(C_1-C_{12})$-Alkoxy, $(C_1-C_{12})$-Alkoxy-$(C_1-C_{12})$-alkyl und $(C_1-C_{12})$-Alkoxy-( $C_1-C_{12}$)-alkoxy steht und

$R^7$ Wasserstoff oder $(C_1-C_4)$-Alkyl bedeutet sowie deren physiologisch verträglichen Salzen, dadurch gekennzeichnet, daß man

a) eine Verbindung der Formel 11

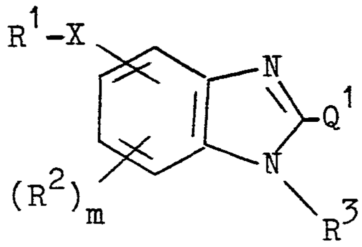

(II)

in welcher m, X, $R^1$, $R^2$ und $R^3$ wie im Anspruch 1 definiert sind und

$Q^1$ i. eine Abgangsgruppe oder

ii. -SH, $-S^-$ oder $-SO_2^-$ bedeutet,

umsetzt mit einer Verbindung der Formel III

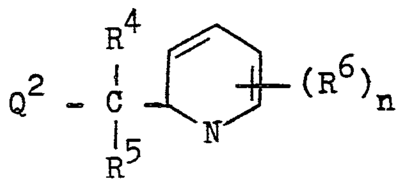

(III)

in welcher n, $R^4$, $R^5$ und $R^6$ wie im Anspruch 1 definiert sind und

$Q^2$ im obengenannten Fall i. -SH, $-S^-$ oder $-SO_2^-$,

im obengenannten Fall ii. eine Abgangsgruppe bedeutet,

b) eine Verbindung der Formel IV

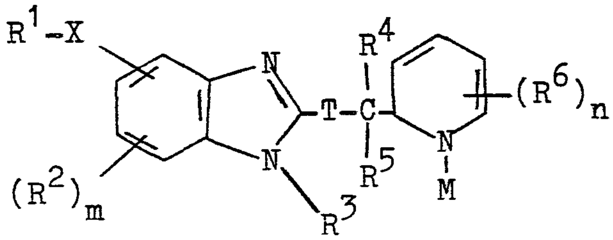

(IV)

in welcher m, n, T, X, $R^1$, $R^2$, $R^3$, $R^4$, $R^5$ und $R^6$ wie im Anspruch 1 definiert sind und

M Wasserstoff, Na, K, Li oder MgHal und Hal Halogen bedeutet,

falls M Wasserstoff bedeutet, oxidiert, andernfalls erhitzt oder hydrolysiert und anschließend oxidiert,

c) eine Verbindung der Formel V

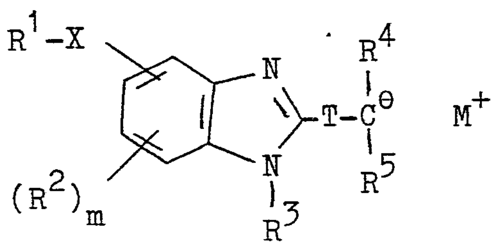

(V)

in welcher m, M, T, X, $R^1$, $R^2$, $R^3$ und $R^4$ wie oben definiert sind

mit einer Verbindung der Formel VI

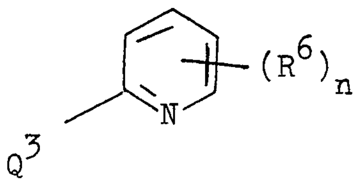

(VI)

in welcher n und $R^6$ wie oben definiert sind und $Q^3$ eine Abgangsgruppe bedeutet, umsetzt, oder

d) eine Verbindung der Formel VII

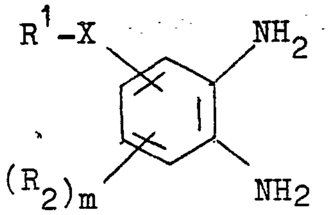

(VII)

in welcher m, X, $R^1$ und $R^2$ wie im Anspruch 1 definiert sind, umsetzt mit einer Verbindung der Formel VIII

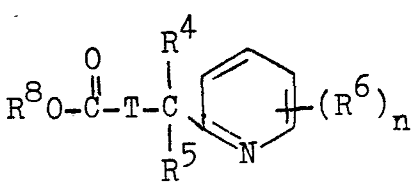

(VIII)

in welcher n, T, $R^4$, $R^5$ und $R^6$ wie in Ansprüch 1 definiert sind und

$R^8$ für eine veresternde Gruppe steht,

in den nach a) -d) erhaltenen Verbindungen der Formel I, in welcher T für -S-oder -SO-und/oder X für -S-oder -SO-steht, gegebenenfalls T und/oder X zur entsprechenden -SO-oder $-SO_2$-Gruppe oxidiert,

eine Verbindung der Formel I, worin $R^3$ für Wasserstoff steht, gegebenenfalls in Position 1 des Benzimidazol-Systems alkyliert oder acyliert,

eine Verbindung der Formel I, worin $R^3$ nicht Wasserstoff oder Alkyl bedeutet, gegebenenfalls verseift und

eine Verbindung der Formel I gegebenenfalls in ihr physiologisch verträgliches Salz überführt, wobei die Reihenfolge der letztgenannten vier Schritte auch umkehrbar ist.

2. Verfahren gemäß Anspruch 1, dadurch gekennzeichnet, daß eine Verbindung der Formel I hergestellt wird und in welcher X $-SO_2-$, $-SO_2-O-$, $-O-SO_2-$, $-SO_2-NR^7-$oder $-NR^7-SO_2-$, T -SO-bedeuten,

$R^2$, $R^3$, $R^4R^5$ und $R^7$ jeweils Wasserstoff bedeuten, mindestens einer der Reste $R^6$ für Was-

serstoff steht und die übrigen Reste wie oben definiert sind, sowie deren physiologisch verträglichen Salze.

3. Verfahren gemäß Anspruch 1 oder 2, dadurch gekennzeichnet, daß eine Verbindung der Formel I hergestellt wird und in welcher der Rest $R^6$ in Position 6 des Pyridin-Rings Wasserstoff bedeutet, sowie deren physiologisch verträglichen Salze.

4. Verfahren gemäß Anspruch 3, dadurch gekennzeichnet, daß eine Verbindung der Formel I hergestellt wird und in welcher der Rest $R^6$ in Position 4 des Pyridin-Rings $(C_1-C_6)$-Alkoxy oder $(C_1-C_6)$-Alkoxy-$(C_1-C_6)$)-alkoxy bedeutet, sowie deren physiologisch verträglichen Salze.

5. Verfahren zur Herstellung eines pharmazeutischen Mittels enthaltend eine oder mehrere Verbindungen der Formel I gemäß einem der Ansprüche 1 -4, dadurch gekennzeichnet, daß man diese Verbindung(en) in eine geeignete Darrreichungsform bringt.

Europäisches Patentamt

# EUROPÄISCHER RECHERCHENBERICHT

Nummer der Anmeldung

EP 86 10 3133

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A-2 134 523 (HÄSSLE)<br>----- | | C 07 D 401/12<br>C 07 D 401/14<br>A 61 K 31/44<br>A 61 K 31/415 |
| | | | **RECHERCHIERTE SACHGEBIETE (Int. Cl.4)** |
| | | | C 07 D 401/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 23-06-1986 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument